# EUROPEAN PATENT SPECIFICATION

(11) **EP 0 546 036 B1**
(45) Date of publication and mention of the grant of the patent: **15.10.1997**
(21) Application number: 91915775.0
(22) Date of filing: 23.08.1991
(51) Int. Cl.: A61K 39/145, A61K 9/127

(54) **VACCINES**
IMPFSTOFFE
VACCINS

(30) Priority: 24.08.1990 GB 9018690
(43) Date of publication of application: 16.06.1993
(73) Proprietor: EVANS MEDICAL LIMITED, Leatherhead, Surrey KT22 7PQ (GB)
(72) Inventor: FORD, Martin, James, Beckenham Kent BR3 3BS (GB)
(74) Representative: Woods, Geoffrey Corlett
(86) International application number: GB9101426
(87) International publication number: WO9203162

(56) References cited:
- EP-A- 0 205 098
- EP-A- 0 356 339
- WO-A-88/08718
- CH-A- 471 896
- FR-A- 2 251 334
- CHEMICAL ABSTRACTS, vol. 91, no. 25, 17 December 1979, Columbus, Ohio, US; abstract no. 206999V, R.LEPRAT ET AL.: 'selective inactivation of hemagglutinin and neuraminidase on mumps virus' page 307 ;column 1 ; &arch.virol.1979,61(4), 273-81 see abstract
- CHEMICAL ABSTRACTS, vol. 97, no. 25, 20 December 1982, Columbus, Ohio, US; abstract no. 212162G, Y.HOSAKA ET AL.: 'hemolysis and fusion by influenza viruses with heat-inactivated neuraminidase activity' page 486 ;column 2 ; &biken j. 1982,25(2),51-62 see abstract
- THE EMBO JOURNAL vol. 6, no. 9, 1987, OXFORD pages 2651 - 2659; T.STEGMANN ET AL.: 'functional reconstitution of influenza virus envelopes' see the whole document document cited in the application

## Description

This invention relates to liposomes, a process for their preparation, and pharmaceutical compositions containing them.

Generally, the poorest responses to influenza vaccines are observed in elderly patients who are most at risk from complications and death following infection with influenza. In addition to these problems, influenza vaccines are unpopular as they are perceived to be ineffective and because of fear of injections.

GB-A-1564500 discloses antigenic preparations containing a plurality of unilamellar microvesicles, otherwise known as virosomes, each microvesicle comprising a single lipid bilayer upon the exterior surface of which is bound an antigenic protein derived from a virus. GB-A-1564500 is related to two U.S. Continuation-in-Part Patents, US-A-4,196,191 and US-A-4,148,876. US-A-4,148,876 discloses antigenic virosome preparations of the type disclosed in GB-A-1564500 in which the antigenic protein is bound by hydrophobic bonding and is a haemagglutinin and neuraminidase sub-unit of a protective surface antigen derived from a myxovirus and having a hydrophobic region.

Hosaka et al, Biken J. 25, 51-62, 1982 reports that influenza virus with heat-inactivated neuraminidase activity had hemolytic activity and was able to induce cell fusion and envelope fusion. The Hosaka et al paper contains no indication that such virus could be used as a vaccine.

EP-A-0 356 339 discloses an influenza immunizing dosage form comprising a liposome, optionally a multilamellar vesicle, and an immunogen of influenza wherein said liposome and immunogen are present in an immunization dose and optionally wherein the immunogen of influenza comprises the haemagglutinin fragment or the bromelain fragment.

WO 88/08718 concerns a method of immunizing against viral infection by administering intranasally an immunogenically effective amount of a viral envelope sub-unit vaccine comprising a glycoprotein complexed with a lipid. The glycoprotein may be the parainfluenza virus glycoprotein HN or F. These glycoproteins are believed to be responsible for, respectively, attachment or haemagglutination and neuraminidase (HN) activities and for progress of infection (F).

CH-A-471896 describes a method for the inactivation of myxoviruses by contacting the viruses in an aqueous suspension at a pH between 6 and 8 in the presence of at least about 0.05% w/v of a non-ionic hydrophilic surface active agent with a fully or partially chlorinated, or chlorinated and fluorinated, lower hydrocarbon which is liquid at room temperature.

We have now found that influenza virosomes which comprise reconstituted virus envelopes and which have been treated to inactivate neuraminidase are highly immunogenic when administered intranasally. significant IgA responses were observed in the lung lavage fluid of mice immunised intranasally but not parenterally. These findings have general applicability. Accordingly, the present invention provides liposomes which have present on their surfaces a polypeptide which is capable of binding to a mucosal cell surface of a human or animal and which renders the liposomes fusogenic, and which are substantially free of active neuraminidase. The liposomes are typically virosomes.

Liposomes are lipid vesicles enclosing a three-dimensional space. Envelope viruses comprise a lipid envelope. Liposomes according to the present invention may therefore be made of the lipid of an envelope virus. The virus envelope may be reconstituted after an envelope virus has been disrupted, for example by a detergent, thereby to form liposomes.

Useful liposomes may also be made of natural or synthetic phosphocholine-containing lipids having one fatty acid chain of from 12 to 20 carbon atoms and one fatty acid claim of at least 8 carbon atoms, for example 12 to 20 carbon atoms. Such lipids include dimyristoylphosphatidylcholine, dioleoylphosphatidylcholine, dipalmitoylphosphatidylcholine, dipalmitoylphosphatidylglycerol, distearoylphosphatidylcholine, phosphatidylcholine, phosphatidylserine and sphingomyelin. Another lipid may also be included in the liposomes, for example cholesterol, which is preferably present as less than 30% w/w of the whole lipid composition. The lipids may further comprise a material to provide a positive or negative charge, such as phosphatidic acid, dicetyl phosphate, phosphatidyl serine or phosphatidyl inositol to provide a negative charge or stearyl amine or other primary amines to provide a positive charge.

The liposomes used in the present invention may be either unilamellar or multilamellar, preferably unilamellar. They are typically biodegradable. The lipid of which they are composed is generally non-antigenic. The liposomes may encapsulate a substance, for example an antibody, antigen or drug. They may therefore be used as a delivery system for the encapsulated component. The liposomes can be used as a general delivery system.

Typically the environment within the liposomes is an aqueous environment. A variety of substances can be encapsulated within the liposomes, such as peptides, proteins or adjuvants. The substance may be a substance against which it is wished to induce an immune response. Substances which may be encapsulated include antigenic subunits prepared from many types of virus such as herpes simplex virus, hepatitis A virus and hepatitis B virus. Proteins or peptides containing class 1 T-cell epitopes may be used. Encapsulation of this material within virosomes may help to generate a cytotoxic T-cell response against them.

The liposomes are preferably in a form which is suitable for intranasal administration. Preferably, therefore, the mucosal cell surface-binding polypeptide imparts on the liposomes the ability to bind to the nasal mucosa or to the mucosa of the lungs. Preferably the diameter of the liposomes is from 5 to 1000nm, for example 10 to 400 nm and most preferably from 20 to 100 nm.

The polypeptide capable of binding to a mucosal cell surface may be glycosylated or unglycosylated. The polypeptide may therefore be in the form of a glycoprotein. The polypeptide renders the liposomes fusogenic so that they are able to fuse with, rather than simply bind to, host cell membranes. These membranes may be either the outer membrane of the host cell or the membrane of endosomes following endocytosis. The polypeptide is typically a virus envelope polypeptide or is derived from a virus envelope polypeptide. All envelope viruses have a surface-binding function. The polypeptide may therefore be a polypeptide which is naturally present on the surface of an envelope virus and which provides the liposomes with the capability of binding to a cell surface. The virus may be a myxovirus such as influenza, mumps or measles virus. In particular the polypeptide may be or be derived from an influenza virus envelope protein, for example of influenza virus type A, B or C.

The polypeptide capable of binding to a cell surface may for example be a haemagglutinin. Haemagglutinin is an integral membrane glycoprotein present in myxoviruses which is commonly composed of three monomers or sub-units. During infection of host cells, it serves two functions. Firstly, it attaches the virus to the cell by the binding of sialic acid residues present on cellular glycoproteins and glycolipids. Second, after internalization of virus into cellular endosomes the subsequent acidification triggers conformational changes in the haemagglutinin which lead to the fusion of viral and cellular membranes. Haemagglutinins are antigenic and stimulate the production of antibodies in hosts.

Another type of polypeptide capable of binding to a cell surface may be a bacterial adhesive protein such as the β-subunit of cholera toxin (CTB) or the heat-labile enterotoxin β-subunit of E. coli (LTB). This may also be used as an adjuvant in combination with haemagglutinin.

Neuraminidase is another glycoprotein which is found as an integral membrane protein in myxoviruses. This functions to cleave sialic acid residues and prevent the irreversible binding of virus to a host cell membrane by haemagglutinin. If active neuraminidase is present in the liposomes, then a significantly lower immunological response is observed. If active neuraminidase would otherwise be present in the liposomes, it must be inactivated. Neuraminidase may be inactivated by heat or by incubation with a neuraminidase inhibitor such as 2,3-dehydro-2-deoxy-N-acetylneuraminic acid (DDAN).

The present liposomes are prepared by a process which comprises forming liposomes which have present on their surfaces a polypeptide which is capable of binding to a mucosal cell surface of a human or animal and which renders the liposomes fusogenic, and which are substantially free of active neuraminidase.

The polypeptide which is capable of binding to a cell surface and which renders the liposomes fusogenic may be added to the lipid materials before, during or after formation of the liposomes. Alternatively, virosomes can be prepared using the natural lipid of the envelope of an envelope virus to provide the necessary lipid component. If the polypeptide does not naturally associate with lipids it may be coupled to a fatty acid such as phosphatidylethanolamine (PE) by the use of a cross-linking agent such as succinimidyl 4-(p-maleimidophenyl)butyrate (SMPB).

Liposomes may for example be prepared by dissolving the lipid starting material in a solvent and evaporating the solvent. The lipid layer is then dispersed with aqueous saline or a buffer (if it is intended to incorporate the polypeptide into the liposomes after vesicle formation) or with an aqueous suspension of the polypeptide (if it is intended to form vesicles in the presence of the polypeptide). The dispersion is then agitated, for example by sonication. Polypeptide may then be added where it is not already incorporated in the surface of the liposomes and the vesicles again agitated.

An alternative method is to add the lipid starting material to an aqueous phase and slowly heat the mixture. It is then agitated to form liposomes. The aqueous phase may contain the polypeptide or it may be added subsequently.

A further method of preparing liposomes comprises the rapid injection of an ethanolic solution of lipid into aqueous saline or a buffer which has previously been purged with nitrogen. The resulting liposome preparation is then concentrated by ultrafiltration with rapid stirring under nitrogen at low pressure to avoid the formation of larger non-heterogeneous liposome. The ethanol may be removed from the vesicle fraction by analysis or washing with an ultra-filter. The polypeptide may be present in aqueous solution or alternatively the liposome fraction obtained after ultrafiltration may be lightly sonicated with the polypeptide.

The liposome preparations obtained in the manner described above comprise aqueous dispersions of the lipid vesicles.

If the liposomes comprise neuraminidase then this must be inactivated. This may be achieved by heating the aqueous dispersion of liposomes comprising active neuraminidase to a temperature of for example from 30 to 60°C, for example from 50 to 60°C, more preferably from 53 to 58°C and most preferably about 55°C. The length of time required for neuraminidase inactivation will depend on the strain of virus and the temperature but is typically from 5 minutes to 5 hours, for example from 15 minutes to 3 hours. At low temperatures eg. 30°C a longer period of heating is required, whilst at higher temperatures a shorter period is required. We have found for influenza virus that heating at 55°C must be for 120 minutes or more, for example up to 180 minutes, in order to achieve an optimum effect. At 56°C, however, the optimal period for heating is from 6 to 10 minutes, for example about 8 minutes.

Alternatively, active neuraminidase may be deactivated by incubation of the liposomes with a neuraminidase inhibitor such as DDAN. As a further alternative active neuraminidase may be inactivated by heat or incubation with a neuraminidase inhibitor prior to incorporation into the liposomes.

A suitable way of preparing liposomes comprises:
(a) disrupting a myxovirus and removing the viral genome and internal viral protein or proteins; and
(b) forming liposomes in the presence of the material remaining, especially the envelope protein or proteins; and
(c) inactivating the neuraminidase present in the thus-formed liposomes.

Step (a) may be achieved be detergent solubilisation of viral particles and removal of internal viral proteins and RNA. In an alternative way of preparing liposomes, the cell surface-binding polypeptide may be prepared by a recombinant DNA methodology. It will then necessarily be provided free of neuraminidase, so liposomes substantially free of active neuraminidase are necessarily obtained.

The liposomes of the present invention may be administered in the form of a pharmaceutical or veterinary composition which additionally comprises a suitable pharmaceutically or veterinarily acceptable carrier or diluent. The compositions are suitable for administration intranasally.

The compositions are preferably provided in a sterilised form. They may take the form of an aerosol. The compositions may further comprise preservatives, stabilisers and other conventional vaccine excipients if required.

The dosage of liposomes will vary depending upon a variety of factors. These include the nature of the cell surface-binding protein, the recipient (human or animal), the vaccination schedule and the extent of adjuvanticity conferred by the preparation. In general a dose of liposomes may be administered intranasally as a single unit or as a multiplicity of a sub-dosage over a period of time. Typically the unit dose for intranasal delivery to a human is from 2 to 500 µg.

The vaccines of the invention exhibit advantages over current influenza vaccines. These include immunogenicity, the convenience of intranasal administration and the production of local mucosal immunity.

The invention will now be further illustrated by means of the following Example. In the accompanying drawings:
Figure 1A shows the ELISA titres against X31 influenza virus in sera from Balb/c mice immunised intranasally (i.n.) with heated and acid-treated virosomes or virus;
Figure 1B shows the ELISA titres against denatured virus in sera from mice immunised i.n. with heated and acid-treated virosomes or virus;
Figure 1C shows the neutralisation titres of sera from mice immunised i.n. with heated and acid-treated virosomes or virus;
Figure 1D shows the HAI titres against virus in sera from mice immunized i.n. with heated and acid-treated virosomes or virus;
Figure 2A shows the ELISA titres against virus in sera from mice immunised i.n. with heated and acid-treated virus;
Figure 2B shows the neutralisation titres against virus in sera from mice immunised i.n. with heated and acid-treated virus;
Figure 3A shows the ELISA titres against virus in sera from mice immunized i.n. with heated and acid-treated virosomes;
Figure 3B shows the neutralisation titres against virus in sera from mice immunised i.n. with heated and acid-treated virosomes;
Figure 4A shows the ELISA titres against virus in sera from mice immunised i.n. with heated and acid-treated virosomes encapsulating the internal proteins and RNA ("virosome-cores");
Figure 4B shows the neutralisation titres against virus in sera from mice immunised i.n. with heated and acid-treated virosome-cores;
Figure 5A shows the ELISA titres against virus in sera from mice immunised i.n. with heated and acid-treated virosomes encapsulating ovalbumin ("ova-virosomes");
Figure 5B shows the neutralisation titres against virus in sera from mice immunised i.n. with heated and acid-treated ova-virosomes;
Figure 6A shows the individual ELISA titres against virus in sera (2 days post-challenge bleed) from mice immunised i.n. with heated and acid-treated virus;
Figure 6B shows the individual neutralisation titres against virus in sera (2 days post-challenge bleed) from mice immunised i.n. with heated and acid-treated virus;
Figure 7A shows the individual ELISA titres against virus in sera (2 days post-challenge bleed) from mice immunised i.n. with heated and acid-treated virosomes;
Figure 7B shows the individual neutralisation titres against virus in sera (2 days post-challenge bleed) from mice immunised i.n. with heated and acid-treated virosomes;
Figure 8A shows the individual ELISA titres against virus in sera (2 days post-challenge bleed) from mice immunised i.n. with heated and acid-treated virosome cores;
Figure 8B shows the individual neutralisation titres against virus in sera (2 days post-challenge bleed) from mice immunised i.n. with heated/acid-treated virosome-cores;
Figure 9A shows the individual ELISA titres against virus in sera (2 days post-challenge bleed) from mice immunised i.n. with heated and acid-treated ova-virosomes;
Figure 9B shows the individual neutralisation titres against virus in sera (2 days post-challenge bleed) from mice immunised i.n. with heated/acid-treated ova-virosomes;
Figure 10A shows ELISA titres showing the effect of heating on the immunogenicity of virosomes administered i.n.;
Figure 10B shows the neutralisation titres showing the effect of heating on the immunogenicity of virosomes administered i.n.;
Figure 11 compares the anti-virus and neutralising antibody response following immunisation with heated virosomes.
Figure 12A shows the effect of heating at 55°C on the immunogenicity of virosomes administered i.n.;
Figure 12B shows the effect of heating at 55°C on the immunogenicity of virosomes administered i.n.;
Figure 13 shows the effect of pre-treating virosomes or mice with gangliosides on the immunogenicity of virosomes given i.n.;
Figure 14A shows the ELISA results of sera from mice immunised i.n. on days 0 and 43 with different doses of influenza virosomes; and
Figure 14B shows the neutralisation results of sera from mice immunised i.n. on days 0 and 43 with different doses of influenza virosomes.

### EXAMPLE

### 1. METHODS

### Preparation of virosomes

The procedure for making the reconstituted virus envelopes was similar to that described by Metsikko et al. (EMBO J. 5, 3429-3435, 1986) and Stegmann et al. (EMBO J. 6, 2651-2659, 1987). A pellet of X31 influenza virus (5mg) was solubilised in 0.7ml of 100mM octaethyleneglycol monododecylether (C₁₂E₈) in dialysis buffer (145mM NaC1, 5mM Hepes, pH 7.4) for 20 min at room temperature. The mixture was centrifuged at 170,000g from 30 min to remove the internal proteins and RNA. 0.56ml of the supernatant was added to 160mg of wet Bio-Beads SM-2 (Trade Mark) and shaken on a rotating table (approx. 400 rpm) for 1 hour at room temperature. The supernatant was removed from the beads with a 23g needle attached to a 1ml syringe and added to 80mg of wet Bio-Beads SM-2 and shaken on a rotating table (approximately 500-600rpm) for 8 min yielding a turbid suspension. The supernatant was removed with a 23g needle and syringe. The virosomes were separated from unincorporated protein by discontinuous sucrose gradients (40%/5% or 40%/20%/5%) spun at 170,000g for 90min. The morphology of the virosomes was analysed by electron microscopy using negative staining with phosphotungstate.

Virosomes containing encapsulated proteins, e.g. ovalbumin (Virosomes + ova), were made as described above except that 100µl of 200 mg/ml ovalbumin was added prior to adding the SM-2 beads. Virosomes-cores were made as described above except that the interal proteins and RNA were not removed by centrifugation.

### ELISA assays

Anti-virus antibodies in the serum from vaccinated mice were measured by ELISA (enzyme-linked immunoadsorbent assay). The virus antigen was diluted in carbonate coating buffer pH 9.5: 1/50 dilution of allantoic fluid from hens eggs inoculated with virus or 1µg/ml of purified egg-grown virus. Microtitre plates were coated with antigen and left at 37°C for 1 hour and then overnight at 4°C. After washing the plates 3 times in 0.05% Tween 20 (Trade Mark) in PBS 100µl of 1% BSA was added and left at 37°C for 1 hour to block the plates. The antisera to be tested was diluted down or across the plate in doubling or half log dilutions in 1% BSA in PBS and left at 4°C overnight. The plates were washed with Tween/PBS before adding the enzyme-conjugated second antibody at 1/500-1/1000 in 1% BSA in PBS. The plates were left at 37°C for 2 hours and washed in Tween/PBS. The substrate, o-phenylenediamine dihydrochloride (OPD) (10mg/100ml) in citrate buffer with 0.01% H₂0₂ was added to the plates and the reaction stopped in H₂SO₄. The plates were read on a microplate reader at 492nm. The titres were end point titres determined by taking the titre at which the OD value was equal to the mean OD value obtained with 1/10 dilution of control normal sera plus 2 standard deviations.

### In Vitro Neutralisation Assay

We have established a microtitre plate-based neutralisation assay on Madin Darby Canine Kidney (MDCK) cells. Serial dilutions of antibody were incubated with 2 logs of virus for 1 hour at 37°C. These were transferred to microtitre plates with 70-90% confluent MDCK cells in Minimum Essential Medium (MEM) media without serum. After incubation at 37°C for 1 hour the supernatant was removed and fresh MEM added with 10µg/ml trypsin. The plates were stained after 48-72 hours and the neutralisation titres read by eye.

### HAI - Haemagglutination inhibition assay

Haemagglutination and haemagglutination inhibition assays were performed as described by Fazekas de St. Groth and Webster, (J. Exp. Med. 124; 331-345, 1966).

### Experiments

Experiments were carried out as follows, referring to the Figures:

### Figure 1

Dose - 5µg of X31 virus/virosomes per mouse in 30µl volume given i.n.
All virosomes were uv. inactivated for 5 min (400µW/cm²) Heating - heating carried out at 55°C for 20 min.
Acid treatment - 1/100th volume of 3M acetate buffer pH 4.8 was added to the virosomes. These were left at 37°C for 15 min before neutralising the acid with 1M Tris pH 7.5.
Second immunization - 6 weeks
Bleed tested - 12 weeks

### Figures 2-5

Dose - 5µg of X31 virus/virosomes per mouse in 30µl volume given i.n.
Virosomes were uv inactivated for 5 min (400µW/cm²)
Heating - heating carried out at 55°C for 20 min.
Acid treatment - 1/100th volume of 3M acetate buffer pH 4.8 was added to the virosomes. These were left at 37°C for 15 min before neutralising the acid with 1M Tris pH 7.5.
Second immunization - 6 weeks

### Figures 6-9

Dose - 5 µg of X31 virus/virosomes per mouse in 30µl volume given i.n.
Virosomes were uv inactivated for 5 min (400µW/cm²)
Heating - heating carried out at 55°C for 20 min.
Acid treatment - 1/100th volume of 3M acetate buffer pH 4.8 was added to the virosomes. These were left at 37°C for 15 min before neutralising the acid with 1M Tris pH 7.5.
Second immunization - 6 weeks
Bleed tested - 12 weeks

### Figure 10

Dose - 3 µg of X31 virosomes per mouse in 30µl volume given i.n.
Virosomes were uv inactivated for 5 min (400µW/cm²)
Heating - heating carried out at 55°C for specified times.
Second immunization - 6 weeks

### Figure 11

Dose - 3 µg of X31 virosomes per mouse in 30µl volume given i.n.
Virosomes were uv inactivated for 5 min (400µW/cm²)
Heating - heating carried out at 55°C for specified times.
Second immunization - 6 weeks
Bleed tested - 8 weeks

### Figure 12

Dose - 3µg of X31 virosomes per mouse in 30µl volume given i.n.
Virosomes were uv inactivated for 5 min (400µW/cm²)
Heating - heating carried out at 55°C for specified times.
Second immunization - 6 weeks
Bleed tested - 12 weeks

### Figure 13

### preincubation with gangliosides and antibody

The virosomes were dialysed against Hepes buffer (145mM NaC1, 5mM Hepes pH 7.4 plus 3mM EDTA). These virosomes were heated at 55°C for 1 hour.
Dose - 3µg/mouse in 30µl volume.
Incubation with gangliosides - Virosomes were incubated at 37°C for 1 hour and then overnight at 4°C with a 12 Molar excess of gangliosides to viral haemagglutinin.
Pretreatment of mice with gangliosides - 100 Molar excess of gangliosides to viral haemagglutinin.
Incubation with antibody - 20µg of virosomes were incubated in 40µg of purified HC2 antibody or 20µg HC2 Fab fragments for 2 hours at 37°C

### Administration of virosomes with CTB

2µg of CTB (B-subunit of cholera toxin) was given together with 3µg of virosomes to each mouse.
Second immunization - 6 weeks
Bleed tested - 12 weeks

### Treatment with DDAN

20µg of virosomes were incubated with 1mM DDAN for 1 hour at 37°C and then at 4°C overnight.
dose per mouse = 3µg in 30µl volume i.n.
Second immunization - 6 weeks
Bleed tested - 12 weeks

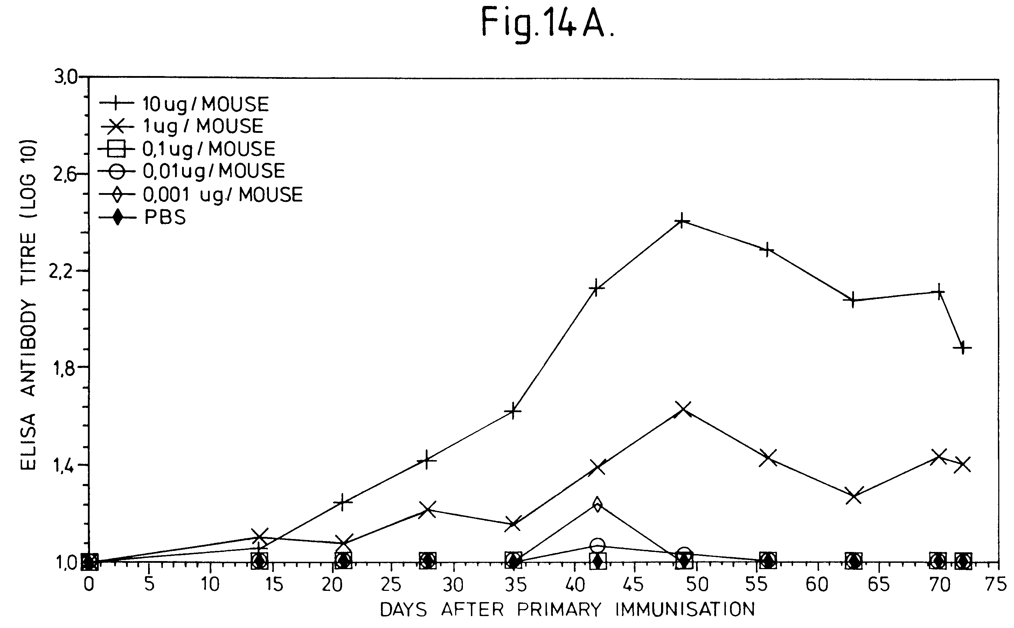

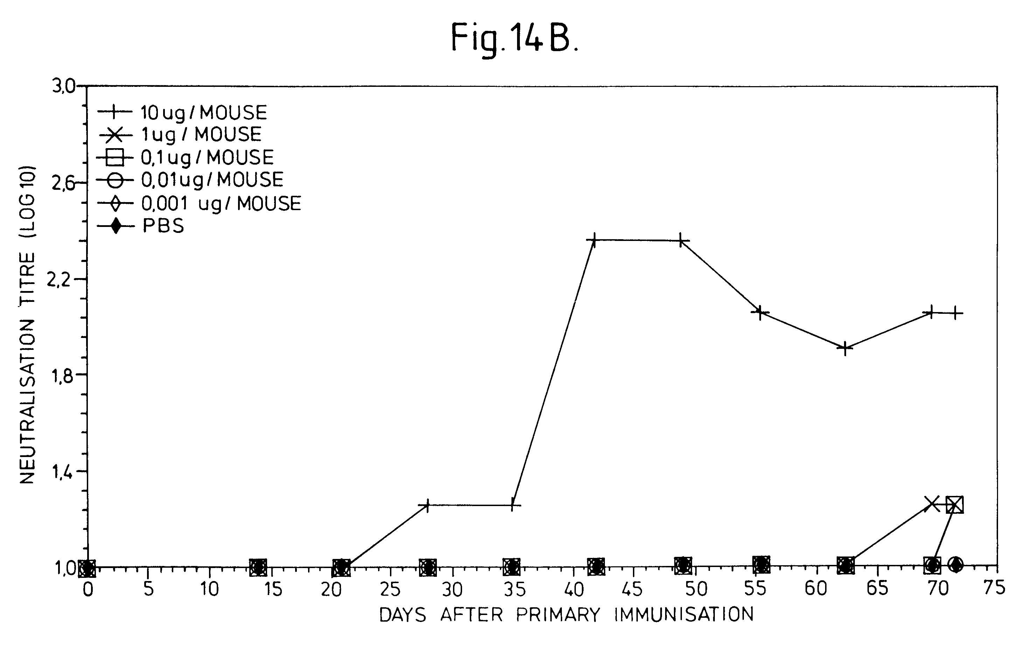

### Figure 14 (Dose response)

Dose - variable dose of X31 virosomes in 30µl volume given i.n.
Virosomes were uv inactivated for 5 min (400µW/cm²)
Heating - heating carried out at 55°C for specified times.
Second immunization - 6 weeks

### 2. RESULTS

### Effect of acid-treatment on the immunogenicity of virus and virosomes

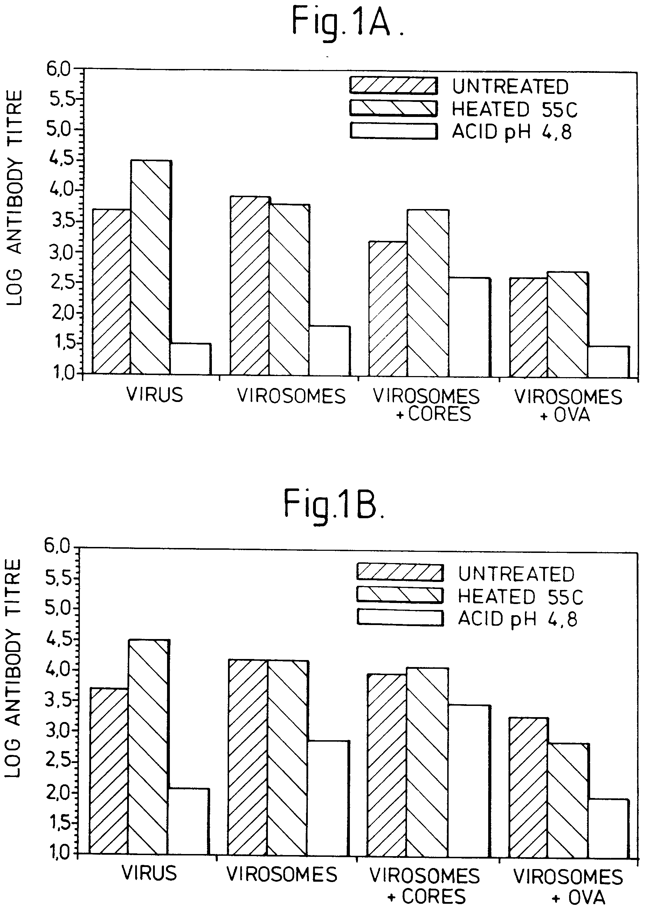

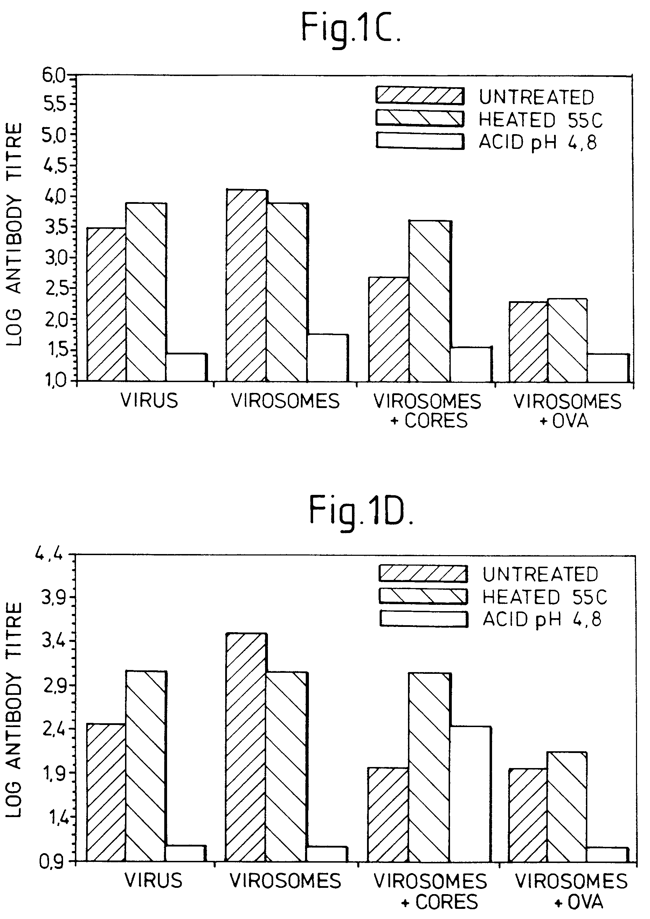

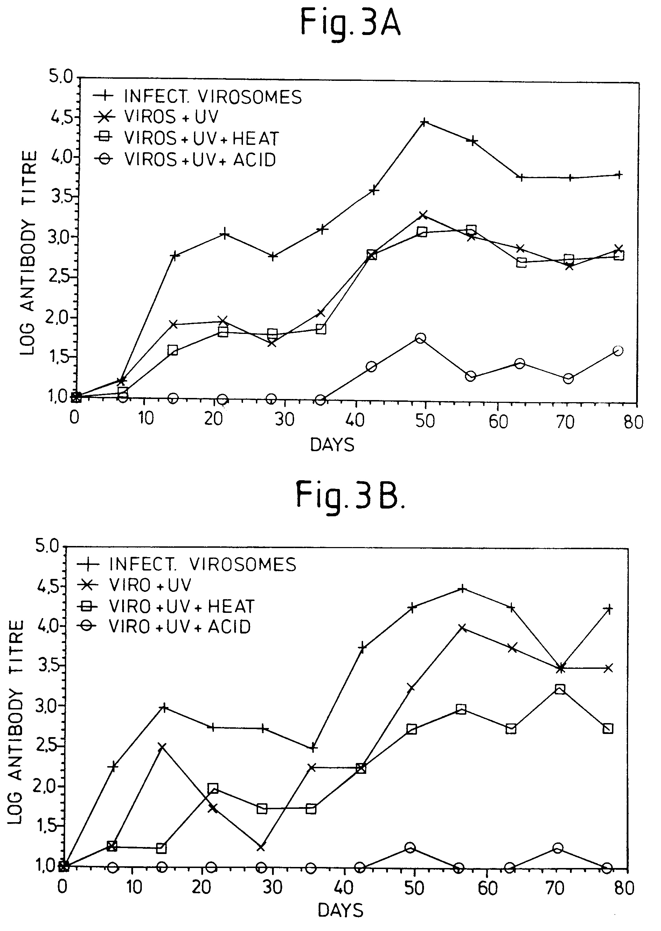

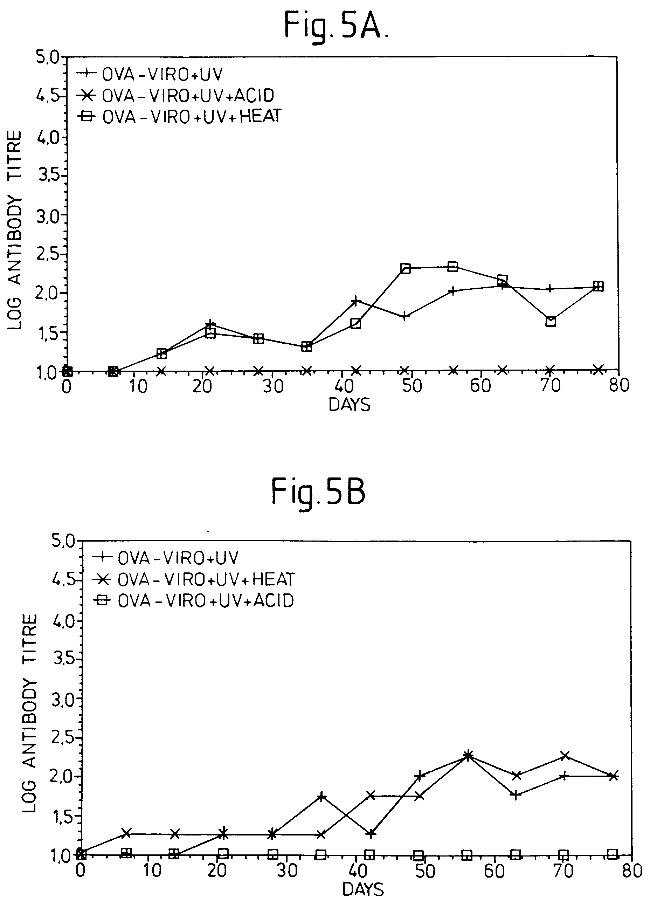

Influenza virus, influenza virosomes, or influenza virosomes containing cores (HBcAg) or ovalbumin were treated with acid (pH 4.8) for 30 min. at 37°C. Acid-treatment of virus or virosomes led to a dramatic reduction in immunogenicity of virus or virosomes given by the intranasal route as assayed by serum ELISA titres against native virus (Figures 1A and 2A-5A). This was not due to the fact that acid destroys some of the neutralisation epitopes on haemagglutinin because lower responses were also observed when the sera were tested against SDS-denatured virus (Figure 1B). In addition, the levels of neutralising antibodies induced were considerably reduced if the inoculum was acid-treated (Figure 1C and 2B-5B).

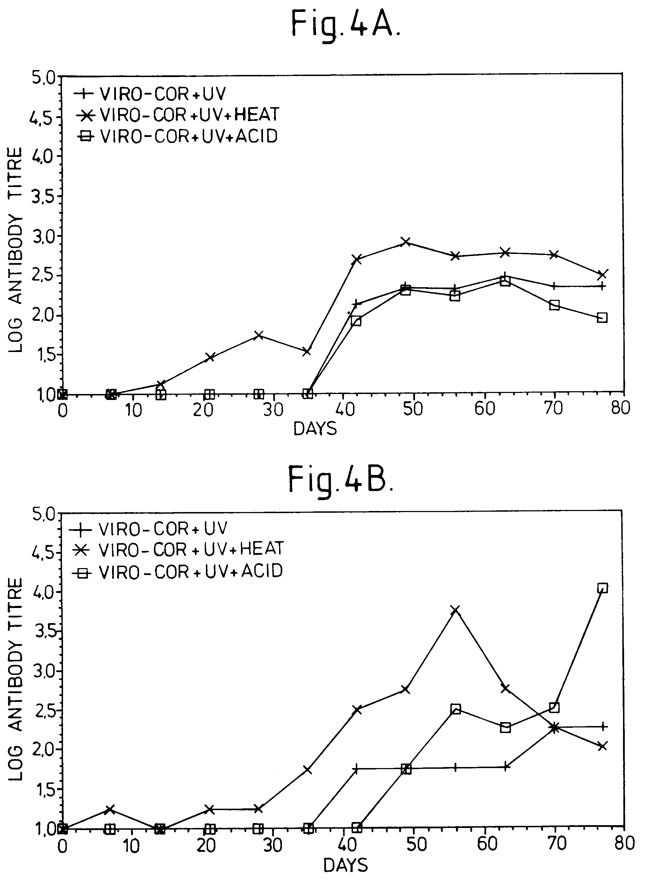

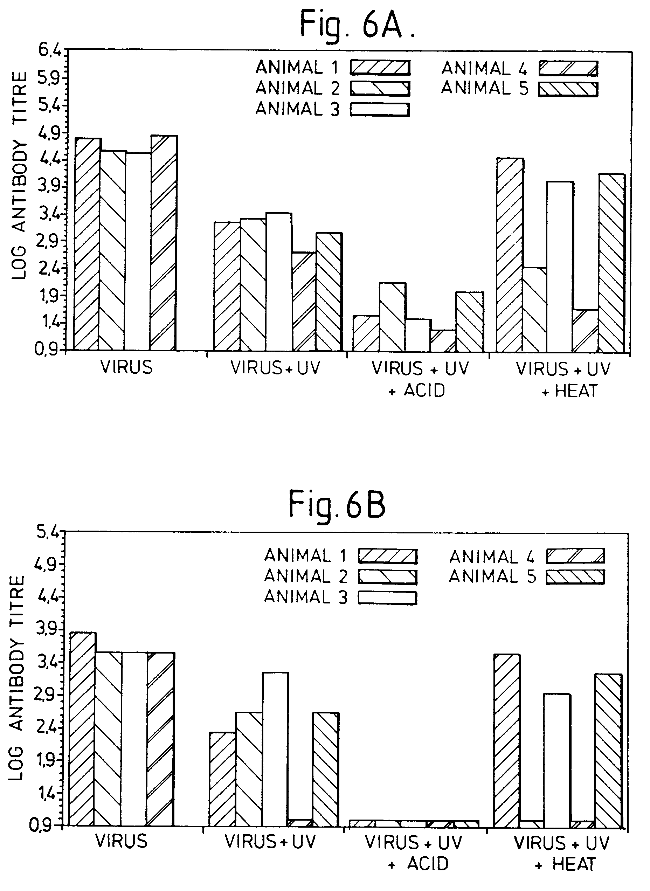

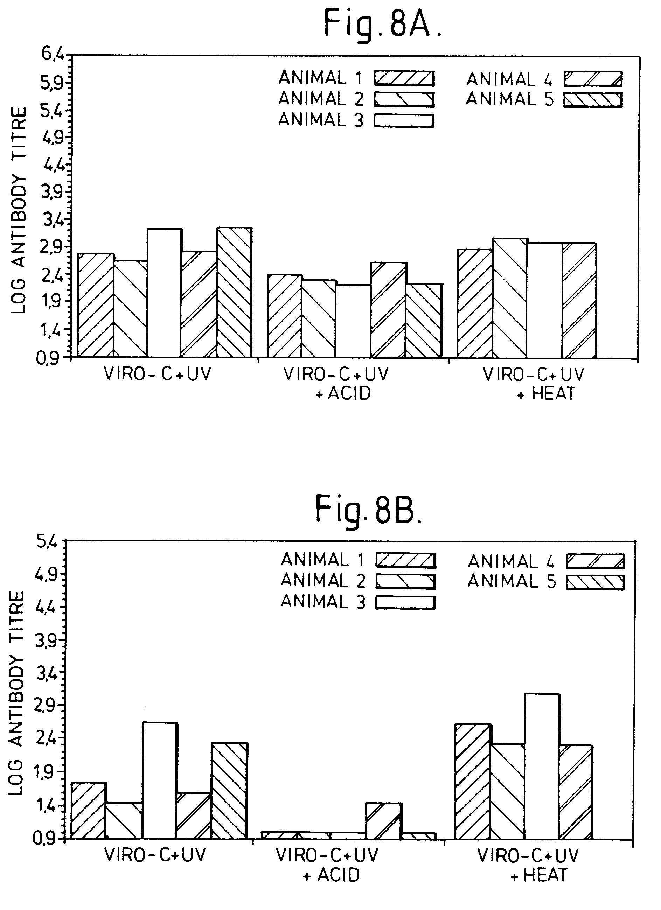

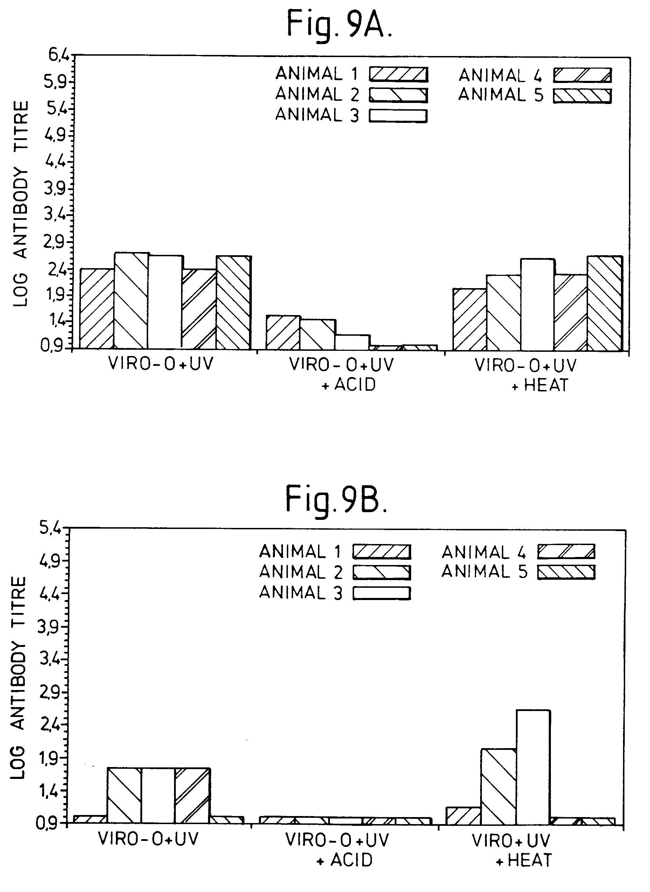

There appeared to be some protection against acid-inactivation of virosomes containing cores but this may be due to insufficient acidification of the boost inoculum (see Figure 4A & B). When the response of individual animals was analyzed there was a consistent reduction in response if the virus or virosomes were acid treated (Figures 6 - 9). We also looked at the haemagglutination inhibition (HI) activity of the sera (Figure 1D), which also show a reduction in the titre of antibody stimulated when virosomes were acid treated before inoculation.

Acid-treatment (pH 4.8) of virus abrogates the ability of virus to fuse with cells while virus attachment is unaffected. This is due to the irreversible conformational shift in the conformation of haemagglutinin that normally occurs inside the endosome after uptake of the virus within coated pits. These results suggest that the virus or virosomes must not only bind to the mucosal surfaces but also fuse with the epithelial cells to stimulate optimal responses.

### Effect of heating at 55°C on the immunogenicity of virus

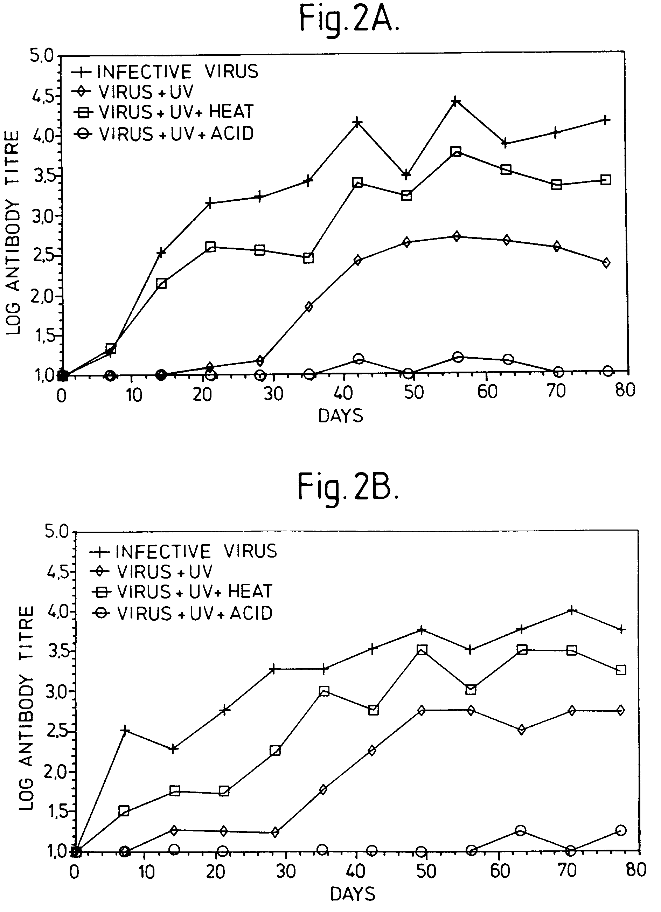

Mice inoculated intranasally with X31 influenza virus heated for 20 min. showed significantly greater serum ELISA, HAI or neutralising antibody titres than mice receiving unheated virus (Figures 1A-D). Both the ELISA titres against native virus and the neutralising titres were approaching those observed following immunization with the same dose of infectious virus (Figure 2). In a further experiment virus was heated for only 8 mins. and again this led to an increase in response following intranasal inoculation (Table 1).

Responses to both the heated or infectious virus were observed at least 21 days before responses to inactivated virus. When the response of individual animals was examined there was an increase in response when the virus was heated and the ELISA titres paralleled the neutralising titres (Figure 6). There was, however, considerable variation probably due to the efficiency of inoculation.

### Effect of heating at 55°C on the immunogenicity of virosomes

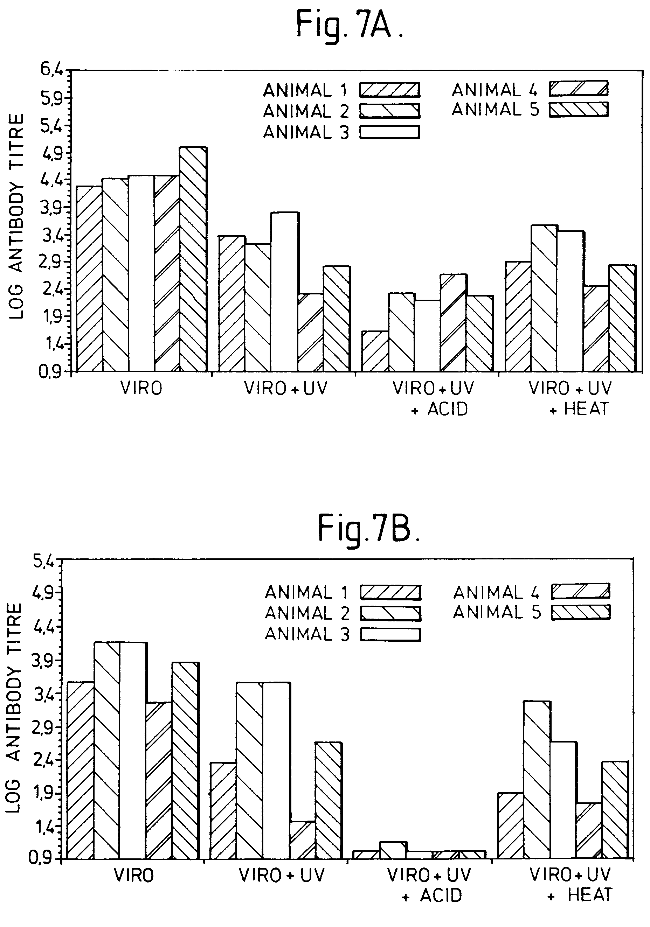

In a preliminary experiment, mice were inoculated intranasally with virosomes, or virosomes containing cores or ovalbumin, that had been heated for 20 min. These heated virosomes stimulated comparable or greater serum ELISA or neutralising titres than mice receiving unheated virosomes (Figures 1-5). Figure 4 shows that uv-inactivated, heated virosomes containing viral cores stimulate a much earlier response than uv-inactivated or acid-treated virosomes. In addition, when the response of individual animals was examined there was little variation within the animal groups (Figure 7). The neutralisation titres showed greater variation but parallelled the ELISA titres. It should be noted that these virosomes were stored at 4°C, so it is possible that much of the neuraminidase activity was lost.

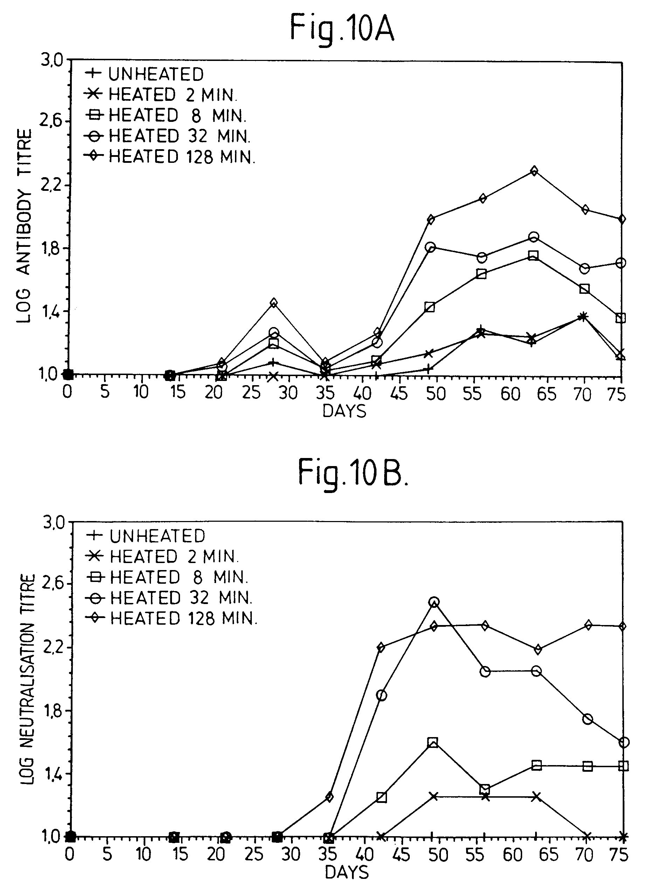

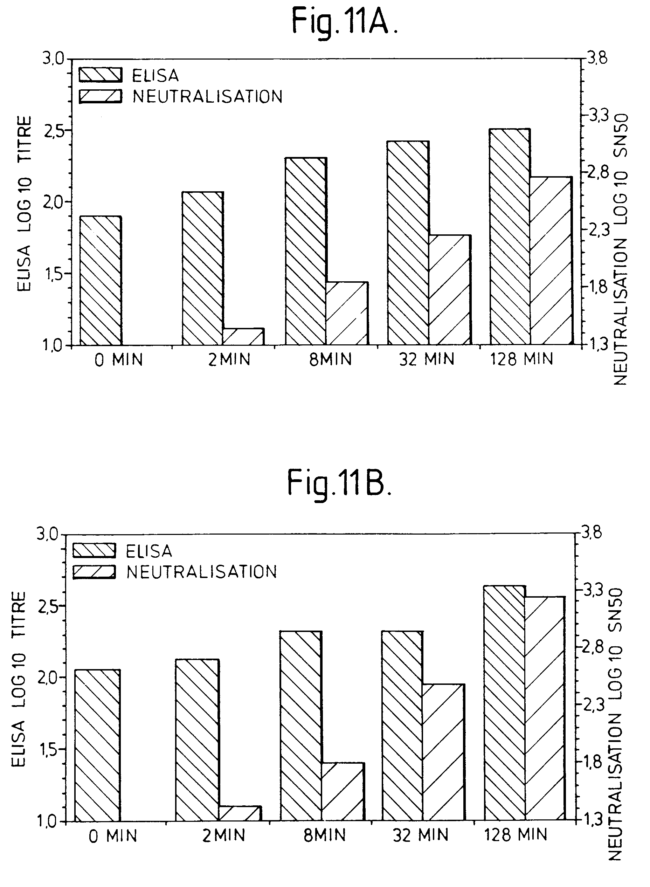

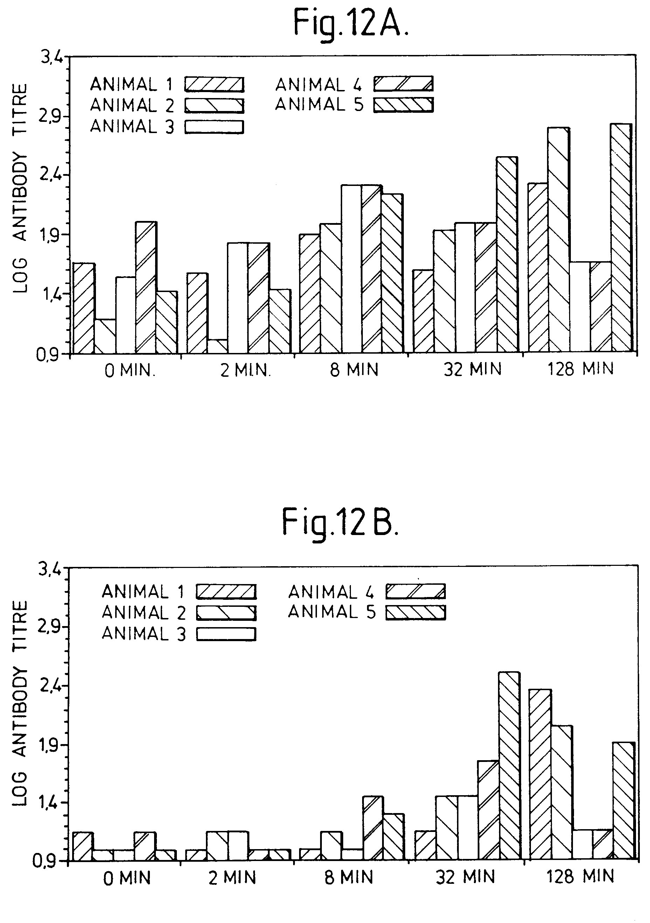

Mice were immunized with fresh virosomes that were stored in 50% glycerol at -20°C. A dramatic increase in immunogenicity was observed if the virosomes were heated for up to 128 mins. (Figures 10A and 11, Table 1). The levels of neutralizing antibodies showed a more dramatic increase with increasing periods of heating (Figures 10B and 11). Animals immunized with unheated virosomes had undetectable levels of neutralising antibodies suggesting that the high responses observed in the previous experiments with unheated virosomes were due to partial inactivation of neuraminidase activity during storage at 4°C. In addition, when serum from individual mice were analyzed a significant increase in ELISA and neutralising antibody titre was observed in sera from mice receiving virosomes heated for increasing periods of time (Table 2, Figure 12).

### Effect of Specific Inactivation of Neuraminidase on the Immunogenicity of Virosomes Given Intranasally

We have carried out an experiment to determine whether the increase in immunogenicity observed with heating of virosomes was due to inhibition of neuraminidase (NA). Thus, the NA in virosomes was specifically inactivated with the neuraminic acid analogue, DDAN. DDAN-treated virosomes stimulated a greater response than untreated virosomes (log titre of 2.2 cf 1.6) showing that inhibition of neuraminidase leads to an increase in immunogenicity of intranasally administered virosomes.

### Effect of Specific Blocking of Virosome Attachment by Pre-incubation with Gangliosides on the Immunogenicity of Virosomes given Intranasally

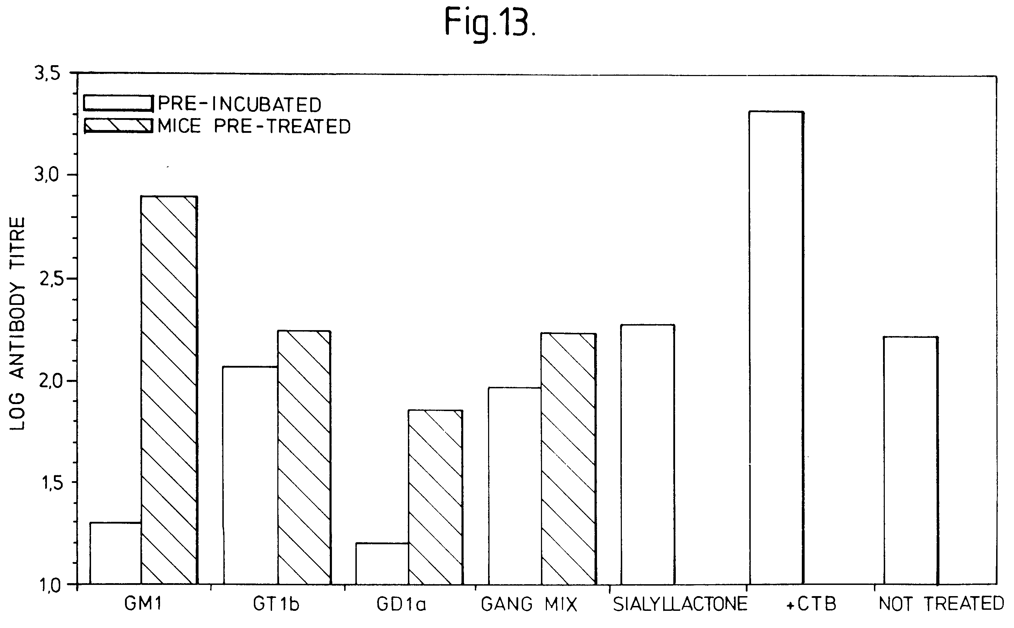

In order to study the effect of blocking virosome attachment on the immunogenicity of intranasally administered virosomes we have pre-incubated virosomes in various sialic acid-containing gangliosides. The immunogenicity of influenza virosomes administered intranasally (i.n.) could be partially abrogated by pre-treating the virosomes with GM1 or GD1a gangliosides but not by pre-treating with GT1b or a ganglioside mixture (Figure 13). Similarly, we have found that the virosome-mediated haemagglutination was partially inhibited only by the GM1 and GB1a gangliosides. These experiments show that binding of virosomes to sialic acid receptors is critical for their immunogenicity.

### Effect of pre-treating mice with gangliosides on the response of mice to virosomes given intranasally

We have studied the effect on the response to virosomes of increasing the viral receptors on the respiratory mucosal surfaces through intranasal pre-treatment of mice with various gangliosides (Figure 13). Pre-treatment with GM1 ganglioside but not GD1a GT1b or a ganglioside mixture led to an increase in response presumably because of an increase in density of receptors or replacement with higher affinity receptors on the mucosal surfaces facilitating greater binding and uptake of the virosomes.

### Effect of Specific Blocking of Virosome Attachment by Pre-incubation with Neutralising Monoclonal Antibodies on the Immunogenicity of Virosomes Given Intranasally

Pre-incubation of virosomes with a neutralising monoclonal antibody (either whole or Fab fragments) completely inhibited haemagglutination. However, the immunogenicity of the virosomes was unaffected by prior incubation of virosomes in whole antibody or Fab fragments or i.n. inoculation of Fab fragments 2 hours after inoculation with untreated virosomes. This result is surprising and may indicate that some neutralising antibodies do not prevent virus binding or entry into mucosal epithelia but some later event (e.g. secondary uncoating). Studying the fate of antibody-treated virus or virosomes should provide some insight into the mechanisms of humoral immunity in the respiratory tract. Moreover, with regards intranasal vaccination of humans, it is encouraging if the presence of local neutralising antibody fails to reduce the immunogenicity of intranasally-administered virosomes.

### Effect of Administering the B-subunit of Cholera Toxin (CTB) Intranasally Together with Virosomes on the Immunogenicity of the Virosomes

We investigated whether CTB could enhance the responses to virosomes administered intranasally. Figure 13 shows that there was a dramatic increase (10-fold) in response to the virosomes when given with CTB).

### Dose response study of virosomes administered intranasally

We have studied the response to a range of doses of virosomes administered intranasally. These virosomes were unheated and fresh so the antibody responses are relatively low. A clear dose response effect was observed with the minimal immunogenic dose being 1µg (Figure 14). We have repeated this experiment using heated virosomes which we would expect to be much more immunogenic.

### Protection against Challenge

All the animals receiving intranasal immunisations of virus or virosomes have been challenged with live virus and the lungs were removed 2 days later. Preliminary challenge lung titres indicate that animals immunised with virus or virosomes that were not acid-treated were completely protected against infection.

**Table 1**

| INTRANASAL IMMUNISATION OF BALB/C MICE WITH INFLUENZA VIROSOMES Effect of heating at 55°C on immunogenicity of virosomes | | | | |
|---|---|---|---|---|
| Group | Antigen | Heat | Dose | ELISA TITRE (OD) |
| 63A | VIROSOMES | Not heated | 3µg | 1.90 (0.25) |
| 63B | VIROSOMES | heated 2min | 3µg | 2.02 (0.25) |
| 63C | VIROSOMES | heated 8min | 3µg | 2.52 (0.43) |
| 63D | VIROSOMES | heated 32min | 3µg | 2.80 (0.54) |
| 63E | VIROSOMES | heated 128min | 3µg | 3.09 (0.64) |
| 63F | VIRUS | Not heated | 3µg | 2.44 (0.44) |
| 63G | VIRUS | heated 8min | 3µg | 2.87 (0.77) |
| 63H | in. PBS | control | | <1.50 (0.17) |
| Bleed tested: 23/2/90 - 4 weeks after primary immunisation | | | | |

**Table 2**

| INTRANASAL IMMUNISATION OF BALB/C MICE WITH INFLUENZA VIROSOMES - RESPONSE OF INDIVIDUAL MICE Effect of heating at 55°C on Immunogenicity | | | | | | |
|---|---|---|---|---|---|---|
| Group Antigen | Heat | ELISA TITRES | | | MEAN TITRE | |
| | | Animal | NV | DV | NV | DV |
| 63A VIROSOMES | Not heated | G | 1.21 | 1.23 | | |
| | | Y | 1.98 | 1.89 | | |
| | | M | 1.26 | 1.14 | 1.84 | 1.44 |
| | | W | 1.48 | 1.23 | | |
| | | R | 1.31 | 1.10 | | |
| 63B VIROSOMES | heated 2min | M | <1.00 | <1.00 | | |
| | | W | 1.38 | 1.02 | | |
| | | G | 2.02 | 1.38 | 1.64 | 1.24 |
| | | R | 1.24 | 1.22 | | |
| | | Y | 1.78 | 1.42 | | |

| Group Antigen | Heat | ELISA TITRES | | | MEAN TITRE | |
|---|---|---|---|---|---|---|
| | | Animal | NV | DV | NV | DV |
| 63C VIROSOMES | heated 8min | R | 2.26 | 1.99 | | |
| | | Y | 1.21 | 1.06 | | |
| | | G | 1.72 | 1.81 | 1.95 | 1.69 |
| | | W | 1.86 | 1.80 | | |
| | | M | 2.12 | 1.13 | | |
| 63D VIROSOMES | heated 32min | M | 2.33 | 1.75 | | |
| | | Y | 2.22 | 2.28 | | |
| | | R | 1.78 | 2.16 | 2.12 | 2.04 |
| | | G | 2.25 | 2.19 | | |
| | | W | 1.65 | 1.07 | | |
| 63E VIROSOMES | heated 128min | R | 2.33 | 2.33 | | |
| | | Y | 2.59 | 2.24 | | |
| | | M | 1.84 | 1.51 | 2.31 | 2.46 |
| | | W | 1.88 | 1.48 | | |
| | | G | 2.47 | 2.99 | | |
| Bleed tested: 30/3/90 - 4 weeks after second immunisation. | | | | | | |

## Claims

1. Liposomes which have present on their surface a polypeptide which is capable of binding to a mucosal cell surface of a human or animal and which renders the liposomes fusogenic, and which are substantially free of active neuraminidase.

2. Liposomes according to claim 1, in which the haemagglutinin is a haemagglutinin of a myxovirus.

3. Liposomes according to claim 2, in which the myxovirus is influenza, mumps or measles virus.

4. Liposomes according to claim 1, in which the polypeptide is a bacterial adhesion polypeptide.

5. Liposomes according to any one of the preceding claims which encapsulate a physiologically active substance.

6. Liposomes according to claim 5, wherein the substance is a peptide, protein or adjuvant.

7. A process for the preparation of liposomes according to any one of the preceding claims, which process comprises forming liposomes which have present on their surfaces the said polypeptide and which are substantially free of active neuraminidase.

8. A process according to claim 7, which comprises:
(a) disrupting a myxovirus and removing the viral genome and internal viral protein or proteins;
(b) forming liposomes in the presence of the material remaining;
(c) inactivating the neuraminidase present in the thus-formed liposomes.

9. A process according to claim 8, wherein the neuraminidase is inactivated by heat or by incubation with neuraminidase inhibitor.

10. A process according to claim 9, in which the inactivation is achieved by heating to a temperature from 50 to 60 °C, or by incubation with 2,3-dehydro-2-deoxy-N-acetylneuraminic acid.

11. A process according to claim 7, wherein liposomes are formed using a said polypeptide which is recombinant polypeptide.

12. A pharmaceutical composition which comprises liposomes according to any one of claims 1 to 6 in association with a pharmaceutically acceptable carrier or diluent.

13. A composition according to claim 12 which is in a form suitable for intranasal administration.

## Patentansprüche

1. Liposomen, welche auf ihrer Oberfläche ein Polypeptid aufweisen, welches fähig zur Bindung an einer Schleimhautzelloberfläche eines Menschen oder eines Tieres ist und welches die Liposomen fusogen macht, und welche im wesentlichen frei von aktiver Neuraminidase sind.

2. Liposomen gemäß Anspruch 1, in denen das Haemaglutinin ein Haemaglutinin eines Myxovirus ist.

3. Liposomen gemäß Anspruch 2, bei welchen es sich bei dem Myxovirus um Influenza-, Mumps- oder Masern-Viren handelt.

4. Liposomen gemäß Anspruch 1, in welchen das Polypeptid ein bakterielles Adhäsions-polypeptid ist.

5. Liposomen gemäß mindestens einem der vorstehenden Ansprüche, welche eine physiologisch aktive Substanz einkapseln.

6. Liposomen gemäß Anspruch 5, worin die Substanz ein Peptid, Protein oder Adjuvans ist.

7. Verfahren zur Herstellung von Liposomen gemäß mindestens einem der vorstehenden Ansprüche, wobei das Verfahren das Bilden von Liposomen umfaßt, welche auf ihrer Oberfläche das Polypeptid aufweisen und welche im wesentlichen frei von aktiver Neuraminidase sind.

8. Verfahren gemäß Anspruch 7, welches folgendes umfaßt:
(a) Aufbrechen eines Myxovirus und Entfernen des viralen Genoms und des/der internen lebenswichtigen Proteins oder Proteine;
(b) Bilden von Liposomen in Gegenwart des verbleibenden Materials;
(c) Inaktivieren der in den derartig gebildeten Liposomen vorhandenen Neuraminidase.

9. Verfahren gemäß Anspruch 8, worin die Neuraminidase durch Wärme oder durch Inkubation mit Neuraminidaseinhibitor inaktiviert wird.

10. Verfahren gemäß Anspruch 9, worin die Inaktivierung durch Erwärmen auf eine Temperatur von 50 bis 60°C oder durch Inkubation mit 2,3-Dehydro-2-desoxy-N-acetylneuraminsäure erreicht wird.

11. Verfahren gemäß Anspruch 7, worin Liposomen unter Verwendung des Polypeptids gebildet werden, bei welchem es sich um ein rekombinantes Polypeptid handelt.

12. Pharmazeutische Zusammensetzung, welche Liposomen nach mindestens einem der Ansprüche 1 bis 6 in Verbindung mit einem pharmazeutisch annehmbaren Träger oder Verdünnungsmittel umfaßt.

13. Zusammensetzung gemäß Anspruch 12, welche in einer für die intranasale Verabreichung geeigneten Form vorliegt.

## Revendications

1. Liposomes qui ont présent sur leurs surfaces un polypeptide qui est capable de se lier à une surface cellulaire de muqueuse d'un humain ou d'un animal et qui rend les liposomes capables de fusionner, et qui sont sensiblement dépourvus de neuraminidase active.

2. Liposomes selon la revendication 1, dans lesquels l'hémagglutinine est une hémagglutinine d'un myxovirus.

3. Liposomes selon la revendication 2, dans lesquels le myxovirus est le virus de la grippe, des oreillons ou de la rougeole.

4. Liposomes selon la revendication 1, dans lesquels le polypeptide est un polypeptide d'adhésion bactérien.

5. Liposomes selon l' une quelconque des revendications précédentes qui encapsulent une substance physiologiquement active.

6. Liposomes selon la revendication 5, dans lesquels la substance est un peptide, une protéine ou un adjuvant.

7. Procédé pour la préparation de liposomes selon l'une quelconque des revendications précédentes, lequel procédé comprend la formation de liposomes qui ont présent sur leurs surfaces ledit polypeptide et qui sont sensiblement dépourvus de neuraminidase active.

8. Procédé selon la revendication 7, qui comprend les étapes consistant :
(a) à rompre un myxovirus et à enlever le génome viral et une protéine ou des protéines virales internes;
(b) à former des liposomes en présence du matériel restant;
(c) à inactiver la neuraminidase présente dans les liposomes ainsi formés.

9. Procédé selon la revendication 8, dans lequel la neuraminidase est inactivée par la chaleur ou par incubation avec un inhibiteur de neuraminidase.

10. Procédé selon la revendication 9, dans lequel l'inactivation est réalisée par chauffage à une température de 50 à 60°C, ou par incubation avec de l'acide 2,3-déhydro-2-désoxy-N-acétylneuraminique.

11. Procédé selon la revendication 7, dans lequel des liposomes sont formés en utilisant ledit polypeptide qui est un polypeptide recombinant.

12. Composition pharmaceutique qui comprend des liposomes selon l'une quelconque des revendications 1 à 6 en association avec un véhicule ou un diluant pharmaceutiquement acceptable.

13. Composition selon la revendication 12, qui est sous une forme appropriée pour l' administration intranasale.
